# EUROPÄISCHE PATENTSCHRIFT

(11) **EP 0 365 981 B1**
(45) Veröffentlichungstag und Bekanntmachung des Hinweises auf die Patenterteilung: **18.01.1995**
(21) Anmeldenummer: 89119292.4
(22) Anmeldetag: 17.10.1989
(51) Int. Cl.: A61B 17/22

(54) **Lithotripsie-Gerät**
Lithotripsy apparatus
Appareil lithotritie

(30) Priorität: 17.10.1988 DE 3835317
(43) Veröffentlichungstag der Anmeldung: 02.05.1990
(73) Patentinhaber: STORZ MEDICAL AG, CH-8280 Kreuzlingen (CH)
(72) Erfinder: Wess, Othmar, Dr., CH-8574 Lengwill-Oberhofen (CH); Hagelauer, Ulrich, Dr., CH-8598 Bottighofen (CH); Heine, Gerold, Dr., D-7772 Uhldingen (CH); Marlinghaus, Ernst, H., Dr., CH-8598 Bottighofen (CH)
(74) Vertreter: Münich, Wilhelm, Dr.

(56) Entgegenhaltungen:
- EP-A- 0 169 311
- EP-A- 0 205 878
- EP-A- 0 257 429
- EP-A- 0 288 698
- Biliary Lithotripsy (Eds. J.T. Ferrucci et al.), Adapted from the Proceedings of the First International Symposium on Biliary Lithotripsy, July 11-13, 1988, Seiten 293-299; J. Pell et al.: "Diasonics, Therasonic Lithotripsy Treatment System"
- ditto: Seiten 253-263; B. Forssman et al.: "Dornier: MPL 9000 Multipurpose Lithotripter"

## Beschreibung

### Technisches Gebiet

Die Erfindung bezieht sich auf ein Lithotripsie-Gerät mit einem Therapie-Schallsender und einer Ortungseinrichtung für Steine, Konkremente etc. sowie einer Patiententisch.

### Stand der Technik

Derartige Lithotripsie-Gerät sind allgemein bekannt.

Wesentliche Voraussetzung für die extrakorporale Lithotripsie von Konkrementen in Lebewesen ist die exakte Positionierung der Konkremente in der räumlich begrenzten Fokuszone, dem sogenannten Fokus des Lithotripters. Alle derzeit bekannten extrakorporalen Lithotripsiegeräte arbeiten entweder mit einer Ultraschall- oder einer Röntgenortungseinrichtung. Insbesondere die Röntgenortungseinrichtung stellt dabei einen kostenmäßig erheblichen Anteil des Gesamtgerätes dar. Die räumliche Anordnung der Röntgenortungseinrichtung in unmittelbarer Nähe der Therapieeinheit reduziert in der Regel wegen nicht optimaler Abbildungsbedingungen die Röntgenqualität. Eine gegenüber vergleichbaren Röntgenanlagen verringert Erkennbarkeit der Konkremente im Körper bzw. eine erschwerte Kontrolle des Zertrümmerungserfolges sind die Folgen.

Das Problem bei den derzeitigen röntgengestützten Geräten für die extrakorporale Lithotripsiek ist, daß die therapeutische Stoß- bzw. Schallwelle über ein Ankopplungsmedium - in der Regel Wasser - in den Körper eingekoppelt wird und dieser Zugang zum Patientenkörper damit für die Röntgenbilderstellung nicht mehr zur Verfügung steht. Unter deutlich anderem Winkel als die Therapiewelle muß man also die bildgebende Röntgenstrahlung den Körper durchlaufen.

Es ist eine Frage des Gerätekonzeptes, ob man der Therapiewelle oder der Röntgenstrahlung die optimale Einstrahlrichtung zuordnet. Entweder für den Zugang der Therapiewelle oder im Hinblick auf die Röntgenbildqualität sind Abstriche zu machen. Dies gilt auch dann, wenn Röntgenanlage und Therapieeinheit speziell aufeinander abgestimmt in einem Gerät integriert angeordnet sind.

Trotz erheblicher konstruktiver Anstrengungen erlauben es die herkömmlichen Geräte für die extrakorporale Lithotripsie nicht, sowohl das Potential einer qualitativ hochwertigen Röntgenabbildung als auch die Möglichkeiten einer günstigen Schallfeldverteilung voll auszunutzen. Insbesondere Schallquellen mit großen Aperturen reduzieren die Schmerzwirkung und lassen trotz ausreichender Zertrümmerungsleistung eine anästhesiefreie Behandlung zu. Gerade diese medizinisch interessanten Quellen sind wegen ihrer großen Ausmasse nur sehr schwer mit einer Röntgenortung zu kombinieren.

Wegen der unterschiedlichen Durchstrahlungsrichtung von Röntgenstrahlen und Therapiewellen entfällt auch die Möglichkeit, das auf dem Ausbreitungsweg der Therapiewelle durchlaufende Körpergewebe auf Hindernisse, Gasblasen etc. zu kontrollieren.

Aus dem Artikel auf Seiten 293-299 in Biliary Lithotripsy (Eds. J.T.Ferrucci et al.), Adapted from the Proceedings of the First International Symposium On Biliary Lithotripsy", July 11-13, 1988, ist ein Lithotripsie-Gerät mit einem Therapie-Schallsender, einer Ortungseinrichtung und einem verstellbaren Patiententisch bekannt. Der Patiententisch weist neben den erforderlichen Freiheitsgraden für die Justierung auch einen Freiheitsgrad auf, mittels dem der Patient zwischen einer Justierposition und einer "Behandlungsposition" verschiebbar ist. Allerdings wird nicht beschrieben, die Freiheitsgrade, die für die Justierung des Patienten erforderlich sind, von den Freiheitsgraden zu trennen, die für die Verschiebung des Patienten zwischen der Justierposition und der Behandlungsposition benötigt werden. Insbesondere wird keine zu dem Patiententisch zusätzliche Zusatzliege mit weiteren Freiheitsgraden, die die Verschiebung des Patienten aus der Justierposition in die Behandlungsposition erlaubt, angegeben.

### Darstellung der Erfindung

Der Erfindung liegt die Aufgabe zugrunde, ein Lithotripsie-Gerät mit einem Therapie-Schallsender und einer Ortungseinrichtung für Steine, Konkremente etc. sowie einer Patiententisch derart weiterzubilden, daß sowohl die Ortungseinrichtung als auch der Schallsender optimal angeordnet sind.

Eine erfindungsgemäße Lösung dieser Aufgabe ist mit ihren Weiterbildungen in den Patentansprüchen gekennzeichnet.

Durch das erfindungsgemäß gestaltete Lithotripsie-Gerät ist es insbesondere möglich, Röntgenanlagen, die für diagnostische und therapeutische Zwecke bereits vorhanden sind oder verwendet werden können, als Ortungseinrichtung und Zertrümmerungskontrolle für einen Lithotripter einzusetzen. Hier sind insbesondere die in der Urologie gebräuchlichen Urologietische von Bedeutung, die auch deswegen im Zusammenhang mit der Lithotripsie von besonderem Interesse sind, weil die Patienten mit ihrer Hilfe in verschiedener Hinsicht auf die eigentliche Lithotripsie vorbereitet werden.

Insbesondere wird erfindungsgemäß eine konsequente räumliche Trennung von Röntgenortung bzw. Röntgenzertrümmerungskontrolle und extrakorporaler Lithotripsie vorgenommen:
Hierzu wird der eigentliche Lithotripter räumlich getrennt, aber in definierter Nachbarschaft zum Röntgensystem aufgestellt. Eine speziell ausgelegte Patientenpositioniereinrichtung sogt dafür, daß der Patient zwischen der Fokuszone der Therapieeinheit und dem Justierzentrum des Röntgensystems hin und her bewegt werden kann.

Ist das zu behandelnde Konkrement durch exakte Positionierung in das Justierzentrum des Röntgensystems gebracht worden, so sorgt eine dem Abstand der Fokuszone des Therapiekopfes zum Röntgen-Justierzentrum entsprechende Translation oder Rotation des Patienten auch für die exakte Positionierung des Konkrementes in der Fokuszone des Lithotripters.

Für eine Zwischenkontrolle der Konkrementposition oder des Zertrümmerungserfolges wird der Patient in den Röntgenstrahlengang zurückbewegt, dort kontrolliert, gegebenenfalls in seiner Position korrigiert und wiederum in die Therapieposition geschwenkt bzw. verschoben.

Unabhängig von der Röntgenposition empfiehlt es sich zur Echtzeitbeobachtung des Zertrümmerungsvorganges bzw. zur Darstellung und Positionierung von röntgennegativen Konkrementen zusätzlich eine Ultraschall-Ortungseinrichtung zu integrieren.

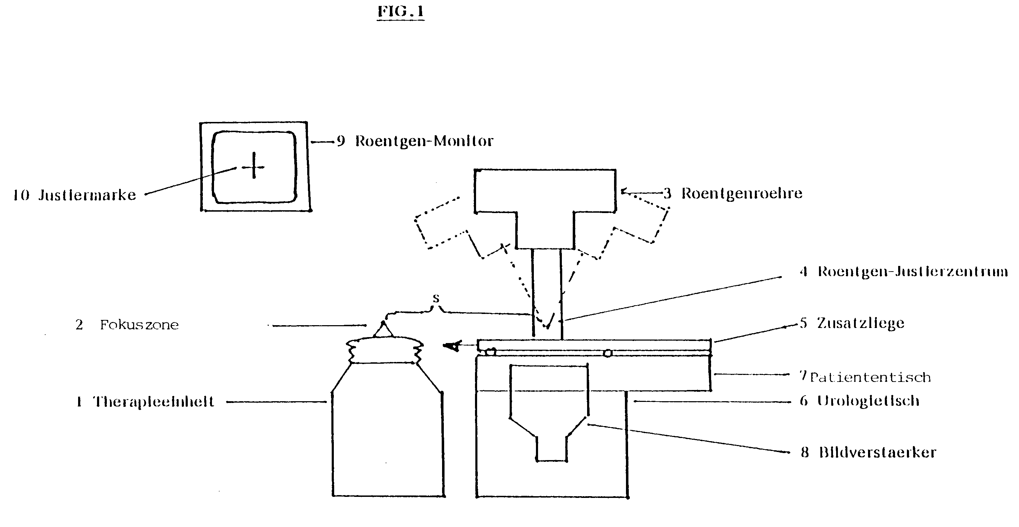

### Kurze Beschreibung der Zeichnung

Die Erfindung wird nachstehend anhand eines Ausführungsbeispiels unter Bezugnahme auf die einzige Figur der Zeichnung näher beschrieben, die als bevorzugte Ausführungsform ein Lithotripsie-Gerät mit einem Röntgen-Urologietisch zeigt.

### Darstellung eines Ausführungsbeispiels

Der eigentliche Lithotripter (1) wird vorteilhafterweise am Fußende eines Urologietisches (6) angeordnet. Der Urologietisch selbst besitzt in der Regel eine höhenverstellbare Patientenlagerung (7). In vielen Fällen ist die Patiententisch zusätzlich horizontal längs und quer verschieblich. Damit sind bereits alle für die Positionierung der Konkremente notwendigen Freiheitsgrade vorhanden. Auf die Patiententisch (7) des Urologietisches wird eine Zusatzliege (5), die lediglich einen Ausschub s mit gerasteten Endpositionen besitzt, abnehmbar montiert. Die Zusatzliege (5) folgt aufgrund der starren Koppelung allen Bewegungen der Patiententisch (7).

Mit der dem Urologietisch eigenen Röntgenanlage (3,8) werden die Konkremente des auf der Zusatzliege (5) gelagerten Patienten auf dem Röntgen-Monitor (9) dargestellt und durch horizontale Verschiebung der Patiententisch (7) mit Justiermarke (10) zur Deckung gebracht.

Besitzt der Urologietisch zusätzlich eine Tomographieeinrichtung kann mit ihrer Hilfe auch die vertikale Position des Konkrementes ermittelt und mit der Vertikalverstellung der Patiententisch (7) auf die vertikale Position des Justierzentrums (4) justiert werden.

Der Ausschub s der Zusatzliege (5) ermöglicht die exakte Übertragung der Konkrementposition aus dem Röntgen-Justierzentrum (4) in die Fokuszone (2) der Therapieeinheit (1), so daß eine Zertrümmerung des Konkrementes erfolgen kann.

Bei gewissen Urologietischmodellen mögen auch die horizontalen Freiheitsgrade der Patiententisch (7) fehlen und nur eine Vertikalverschiebung des Patienten möglich sein. In diesem Fall lassen sich die benötigten horizontalen Freiheitsgrade in die Zusatzliege (5) integrieren, so daß auch dann eine Justierung der Konkremente in das Justierzentrum (4) und ein anschließende positionsgenaue Verlagerung in die Fokuszone (2) möglich wird.

Das Verfahren ist auch dann anwendbar, wenn der Urologietisch keine Tomographieeinrichtung besitzt und nur die horizontale Position der Konkremente mittels der Röntgenanlage (3,8) ermittelt werden kann. In diesem Fall dient die Röntgenanlage (3,8) zur horizontalen Justierung und Beurteilung der Konkrementbeschaffenheit bzw. des Zertrümmerungserfolges.

Der Lithotripsieteil (1) wird vorteilhafterweise so am Fußende des Urologietisches (6) angeordnet, daß die Möglichkeit besteht, ihn in eine Parkposition zu verlagern. Auf diese Weise läßt sich die freie Zugänglichkeit des Urologietisches gewährleisten für den Fall, daß nicht oder erst später lithotripsiert wird.

## Patentansprüche

1. Lithotripsie-Gerät mit
- einem Therapie-Schallsender, dessen Schallwellen in einer Fokuszone (2) fokussiert sind,
- einer Ortungseinrichtung für Steine, Konkremente etc., die beabstandet von dem Therapie-Schallsender angeordnet ist, und
- einem Patiententisch (7), auf dem eine Zusatzliege (5) angeordnet ist, die derart verstellbar ist, daß der zu zertrümmernde Stein bzw. das zu zertrümmernde Konkrement unter Beobachtung mit der Ortungseinrichtung in eine Justierposition überführbar ist und daß die Zusatzliege (5) gegenüber dem Patiententisch (7) in eine Behandlungsposition bewegbar ist,
dadurch **gekennzeichnet**, daß der Patiententisch (7) gemeinsam mit der Zusatzliege (5) derart verstellbar ist, daß der zu zertrümmernde Stein bzw. das zu zertrummernde Konkrement unter Beobachtung mit der Ortungseinrichtung in ein Justierzentrum überführt wird, das von der Fokuszone (2) des Therapie-Schallsenders einen bestimmten Abstand hat, und daß durch die Bewegung der Zusatzliege (5) gegenüber dem Patiententisch (7) um den besagten Abstand der Stein bzw. das Konkrement aus dem Justierzentrum (4) in die Fokuszone (2) des Therapie-Schallsenders überführt wird.

2. Gerät nach Anspruch 1,
dadurch **gekennzeichnet**, daß die Bewegung von der Justierposition in die Behandlungsposition eine horizontale Verschiebung in wenigstens einer Richtung ist.

3. Gerät nach Anspruch 1,
dadurch **gekennzeichnet**, daß die Bewegung von der Justierposition in die Behandlungsposition eine Drehung ist.

4. Gerät nach einem der Ansprüche 1 bis 3,
dadurch **gekennzeichnet**, daß die Ortungseinrichtung aus einem Röntgen-C-Bogen besteht.

5. Gerät nach einem der Ansprüche 1 bis 3,
dadurch **gekennzeichnet**, daß die Ortungseinrichtung eine Röntgenanlage ist, die in einen Röntgentisch bzw. Urologietisch (6) integriert ist, der als Patiententisch (7) dient.

6. Gerät nach einem der Ansprüche 1 bis 5,
dadurch **gekennzeichnet**, daß die Zusatzliege (5) auf dem Patiententisch (7) abnehmbar montiert ist

7. Gerät nach einem der Ansprüche 1 bis 6,
dadurch **gekennzeichnet**, daß die Zusatzliege (5) zwei horizontale Justierfreiheitsgrade besitzt.

8. Gerät nach einem der Ansprüche 1 bis 7,
dadurch **gekennzeichnet**, daß der Therapie-Schallsender mit einer zusätzlichen Ultraschall-Ortungseinheit ausgestattet ist.

9. Gerät nach einem der Ansprüche 1 bis 8,
dadurch **gekennzeichnet**, daß der Therapie-Schallsender in eine Parkposition bewegt werden kann.

10. Gerät nach einem der Ansprüche 1 bis 9,
dadurch **gekennzeichnet**, daß der Therapie-Schall-Sender um die Fokuszone (2) herum isozentrisch drehbar gelagert ist.

## Claims

1. Lithotripter apparatus comprising
- a therapeutic sonic emitter whose sonic waves are focused in a focusing zone (2),
- a means for detecting calculi, concrements, etc. which is disposed at a spacing from said therapeutic sonic emitter, and
- a patient-supporting table (7) carrying an additional couch (5) which is adapted to be so adjusted that the calculus or the concrement, respectively, to be fragmented may be displaced into an adjustment position, under observation by means of said detecting means, and that said additional couch (7) may be moved relative to said patient-supporting table (7) into a treatment position,
**characterized** in that said patient-supporting table (7) is adapted to be adjusted, together with said additional couch (5), in such a way that the calculus or the concrement, respectively, to be fragmented will be displaced into an adjustment center, under observation by means of said detecting means, which adjustment center is arranged at a defined spacing from the focusing zone (2) of said therapeutic sonic emitter, and that as a result of the movement of said additional couch (5) relative to sold patient-supporting table (7) by said spacing, the calculus or concrement, respectively, is moved out of the adjustment center (4) into the focusing zone (2) of said therapeutic sonic emitter.

2. Apparatus according to Claim 1,
**characterized** in that the movement from the adjustment position into the treatment position is a horizontal displacement in at least one direction.

3. Apparatus according to Claim 1,
**characterized** in that the movement from the adjustment position into the treatment position is a rotation.

4. Apparatus according to any of Claims 1 to 3,
**characterized** in that said detecting means consists of an X-ray C-arch.

5. Apparatus according to any of Claims 1 to 3,
**characterized** in that said detecting means is an X-ray system integrated into an X-ray table or urologic table (6), respectively, which serves as patient-supporting table (7).

6. Apparatus according to any of Claims 1 to 5,
**characterized** in that said additional couch (5) is detachably mounted on said patient-supporting table (7).

7. Apparatus according to any of Claims 1 to 6,
**characterized** in that said additional couch (5) presents two degrees of freedom for horizontal adjustment.

8. Apparatus according to any of Claims 1 to 7,
**characterized** in that said therapeutic sonic emitter is equipped with an additional ultrasonic detecting means.

9. Apparatus according to any of Claims 1 to 8,
**characterized** in that said therapeutic sonic emitter is adapted for movement into a parking position.

10. Apparatus according to any of Claims 1 to 9,
**characterized** in that said therapeutic sonic emitter is supported for isocentric rotation about said focusing zone (2).

## Revendications

1. Appareil lithotriteur comprenant
- un émetteur sonique thérapeutique dont les ondes sonores sont concentrées dans une zone de focalisation (2),
- des moyens à détecter des calculs, concrétions, etc., qui sont espacés par une disance dudit émetteur sonique thérapeutique, et
- une table à porter le patient (7), qui portent un lit supplémentaire (5) configuré pour être ajusté de façon que le calcul ou respectivement la concrétion à broyer puissent être déplacé dans une position d'ajustement, en l'observant moyennant lesdits moyens détecteurs, et que ledit lit supplémentaire (7) puisse être porté, relativement à ladite table à porter le patient (7), dans une position de traitement,
**caractérisé** en ce que ladite table à porter le patient (7) est configurée pour être ajustée, ensemble avec ledit lit supplémentaire (5), de façon que le calcul ou respectivement la concrétion à broyer soient déplacé dans un centre d'ajustement, en l'observant moyennant lesdits moyens détecteurs, ledit centre d'ajustement étant disposé à une distance définie de la zone de focalisation (2) dudit émetteur sonique thérapeutique, et de façon qu'en conséquence du mouvement dudit lit supplémentaire (5) relatif à ladite table à porter le patient (7) par ladite distance, le calcul or respectivement la concrétion soit éloigné dudit centre d'ajustement 84) dans la zone de focalisation (2) dudit émetteur sonique thérapeutique.

2. Appareil selon la revendication 1,
**caractérisé** en ce que le mouvement de la position d'ajustement dans la position de traitement est un déplacement horizontal en au moins une direction.

3. Appareil selon la revendication 1,
**caractérisé** en ce que le mouvement de la position d'ajustement dans la position de traitement est une rotation.

4. Appareil selon une quelconque des revendications 1 à 3,
**caractérisé** en ce que lesdits moyens détecteurs sont formés par un arc en C à rayonnement X.

5. Appareil selon une quelconque des revendications 1 à 3,
**caractérisé** en ce que lesdits moyens détecteurs sont un système à rayonnement X intégré dans une table à l'examen par rayonnement X ou respectivement une table urologique (6), qui sert comme table à porter le patient (7).

6. Appareil selon une quelconque des revendications 1 à 5,
**caractérisé** en ce que ledit lit supplémentsaire (5) est monté, de façon démontable, sur ladite table à porter le patient (7).

7. Appareil selon une quelconque des revendications 1 à 6,
**caractérisé** en ce que ledit lit supplémentaire (5) présente deux degrés de liberté pour l'ajustement horizontal.

8. Appareil selon une quelconque des revendications 1 à 7,
**caractérisé** en ce que ledit émetteur sonique thérapeutique est prévu des moyens détecteurs aux ultrasons supplémentaires.

9. Appareil selon une quelconque des revendications 1 à 8,
**caractérisé** en ce que ledit émetteur sonique thérapeutique est conçu pour un mouvement dans une position de parking.

10. Appareil selon une quelconque des revendications 1 à 9,
**caractérisé** en ce que ledit émetteur sonique thérapeutique est logé pour une rotation isocentrique autour de ladite zone de focalisation (2).
